Europäisches Patentamt

European Patent Office

Office européen des brevets

(11) Veröffentlichungsnummer: **0 370 124 B1**

(12) # EUROPÄISCHE PATENTSCHRIFT

(45) Veröffentlichungstag der Patentschrift: **14.07.93**

(51) Int. Cl.<sup>5</sup>: **A61B 6/03**, H05G 1/52

(21) Anmeldenummer: **88119417.9**

(22) Anmeldetag: **22.11.88**

(54) **Computer-Tomograph.**

(43) Veröffentlichungstag der Anmeldung:
**30.05.90 Patentblatt 90/22**

(45) Bekanntmachung des Hinweises auf die
Patenterteilung:
**14.07.93 Patentblatt 93/28**

(84) Benannte Vertragsstaaten:
**DE FR GB**

(56) Entgegenhaltungen:
**EP-A- 0 282 870**
**DE-A- 2 811 464**

**APPLIED OPTICS, Band 24, Nr. 23, Dezember
1985, New York/US, K.R. Peschmann u.a.
"High-speed computed tomography: systems and performance", SS. 4052-4060**

(73) Patentinhaber: **SIEMENS AKTIENGESELL-
SCHAFT
Wittelsbacherplatz 2
W-8000 München 2(DE)**

(72) Erfinder: **Schwierz, Günter, Dr. rer. nat.
Rödlaser Strasse 9
W-8524 Neunkirchen(DE)**
Erfinder: **Kestler, Joachim, Dipl.-Ing.
Judengasse 4
W-8551 Pretzfeld(DE)**

**Beschreibung**

Die Erfindung betrifft einen Computertomographen mit einem Röntgenstrahler mit kreisförmigem, eine Meßöffnung umschließendem Anodenbogen, auf dem der Fokus zur Durchstrahlung eines in der Meßöffnung liegenden Objektes mit einem fächerförmigen Röntgenstrahlenbündel unter verschiedenen Richtungen mit Hilfe eines Ablenksystems bewegt wird, sowie mit einem kreisförmigen Strahlendetektor, der aus einer Reihe von Detektorelementen besteht, deren Meßwerte über ein Datenerfassungssystem einem Rechner zugeführt werden, welcher die Schwächungswerte vorbestimmter Punkte der untersuchten Objektschicht berechnet und deren bildliche Wiedergabe bewirkt.

Bei Computertomographen mit mechanisch bewegtem Röntgenstrahler zur Durchstrahlung des Objektes unter verschiedenen Richtungen ist zur Erzielung artefaktarmer Bilder des schlagenden Herzens die Sammlung der Meßwerte während mehrerer Herzzyklen erforderlich. Die Meßwerte werden dabei immer bei derselben Herzphase erzeugt. Zur Überwachung der unvermeidbaren Schwankungen der Hochspannung an dem Röntgenstrahler sind bei diesen Computertomographen Monitordetektoren mechanisch mit dem Röntgenstrahler verbunden, werden mit diesem also mitgeführt. Auf diese Weise ist es möglich, diese Schwankungen, die zu Änderungen der Meßwerte führen, rechnerisch zu berücksichtigen.

Ein Computertomograph der eingangs genannten Art ist in US 4 352 021 beschrieben. Bei diesem Computertomographen ist eine sehr schnelle Bewegung des Fokus auf dem Anodenbogen möglich, so daß die Aufnahme von Herzbewegungsphasen während eines einzigen Herzzyklus möglich ist. Der Fokus eines Elektronenstrahles wird dabei mit Hilfe eines elektrischen und/oder magnetischen Ablenksystems über den kreisförmigen Anodenbogen geführt. Mechanische Teile zur Bewegung des Fokus sind demgemäß nicht mehr vorhanden. Deshalb ist auch die mechanische Mitführung von Monitordetektoren ausgeschlossen.

Der Erfindung liegt die Aufgabe zugrunde, einen Computertomographen der eingangs genannten Art so auszubilden, daß Meßwertschwankungen, die von Schwankungen der Hochspannung des Röntgenstrahlers herrühren, nicht zu Bildartefakten führen.

Diese Aufgabe ist erfindungsgemäß dadurch gelöst, daß das Ablenksystem und das Datenerfassungssystem derart synchronisiert sind, daß bei jedem der n Umläufe des Fokus die Fokusbewegung und das Auslesen der Meßwerte gegenüber dem vorausgegangenen Umlauf relativ zur Rippelperiode der Hochspannung des Röntgenstrahlers geringfügig phasenversetzt erfolgt.

Bei der Erfindung ist davon ausgegangen, daß

- bei den zur Anwendung kommenden Hochspannungsgeneratoren die Schwankung der Hochspannung, der sogenannte Spannungsrippel, weitgehend periodisch erfolgt, wobei die Rippelfrequenz ein bekanntes Vielfaches der Netzfrequenz ist, und
- zur Erzielung rauscharmer Bilder der Fokus wiederholt den Anodenbogen überstreichen muß.

Dabei ist es gleichgültig, ob vor der Bildrekonstruktion homologe Meßdaten zusammengefaßt werden oder die zu den einzelnen Umläufen des Fokus gehörigen CT-Bilder superponiert werden.

Die Erfindung ist nachfolgend anhand eines in der Zeichnung dargestellten Ausführungsbeispieles näher erläutert. Es zeigen:

Fig. 1    einen Computertomographen nach der Erfindung, und

Fig. 2    eine Einzelheit des Computertomographen gemäß Fig. 1.

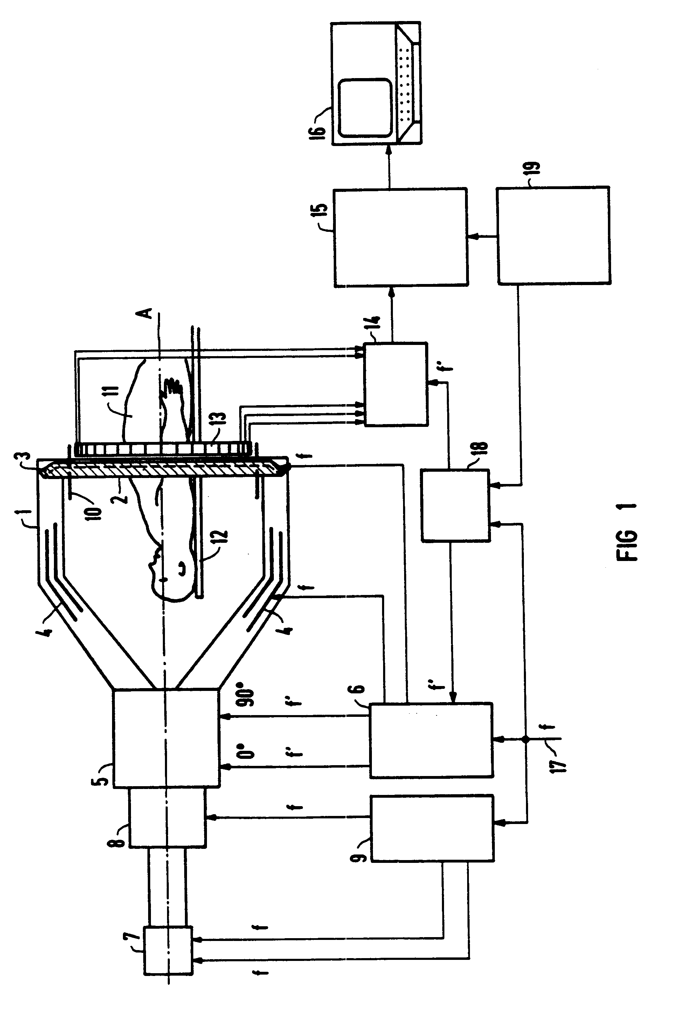

In der Fig. 1 ist ein Computertomograph mit einem Vakuumgefäß 1 eines Röntgenstrahlers dargestellt, in dem ein eine Meßöffnung 2 umschließender, kreisförmiger Anodenbogen 3 liegt. Der von einem Elektronenstrahl erzeugte Fokus auf dem Anodenbogen 3 beschreibt auf diesem eine Kreisbahn. Hierzu wird der Elektronenstrahl mit Hilfe eines gekrümmten Ablenkkondensators 4 und einer Ablenkanordnung 5, der ein Ablenkgenerator 6 zugeordnet ist, abgelenkt. Der Röntgenstrahler besitzt eine Elektronenkanone 7 für die Erzeugung des Elektronenstrahles, der ein, einen Fokus gewünschter Größe auf dem Anodenbogen 3 erzeugender, rotierender Strahl ist. Eine Fokussierungsanordnung 8 bewirkt die Fokussierung des Elektronenstrahles. Der Röntgenstrahler 1, 3, 5, 7, 8 wird von einem Hochspannungsgenerator 9 mit Hoch- und Heizspannung versorgt.

Der auf der Anodenbahn 3 rotierende Fokus erzeugt ein mit Hilfe einer Blende 10 eingeblendetes, fächerförmiges, rotierendes Röntgenstrahlenbündel, das einen Patienten 11, der auf einer Liege 12 in der Meßöffnung 2 liegt, unter verschiedenen Richtungen durchstrahlt und nach dem Austritt aus dem Patienten 11 auf einem kreisförmigen Strahlendetektor 13, der aus einer Reihe von Detektorelementen besteht, auftrifft. Es verläuft hierzu unter einem von 90° abweichenden Winkel zur Achse A. Die Meßwerte der Detektorelemente werden über ein Datenerfassungssystem 14 einem Rechner 15 zugeführt, welcher die Schwächungswerte vorbestimmter Punkte der untersuchten Schicht des Patienten 11 berechnet und deren bildliche Wiedergabe auf einem Auswertesystem 16 bewirkt.

2

Die Netzspannung liegt an einer Leitung 17 und deren Frequenz wird von einer Synchronisiereinrichtung 18 für das Ablenksystem 6 und das Datenerfassungssystem 14 in der Weise ausgewertet, daß bei jedem der n Umläufe des Fokus die Fokusbewegung und das Auslesen der Meßwerte gegenüber dem vorausgegangenen Umlauf relativ zur Rippelperiode der Hochspannung des Röntgenstrahlers, die von der Netzfrequenz abgeleitet ist, geringfügig phasenversetzt erfolgt. Hierzu ist der Synchronisiereinrichtung 18 und dem Rechner 15 noch ein Steuerrechner 19 zugeordnet. Die Netzfrequenz ist in der Regel von der Rippelfrequenz verschieden, welche ein bekanntes, ganzzahliges Vielfaches der Netzfrequenz ist.

Der Phasenversatz wird als das p-fache des n-ten Teiles der Rippelperiode gewählt. Dabei bedeuten n die Anzahl der verwendeten Fokusumläufe und p eine beliebige natürliche Zahl. Vorzugsweise wird p so gewählt, daß p und n teilerfremd sind. Die Wirksamkeit der Methode ist wie folgt zu erkennen:

r1 (f x t) bezeichne die vom Hochspannungsgenerator 9 gelieferte Rippelfunktion mit der Rippelfrequenz f und der Zeitvariablen t.

Die Fourier-Entwicklung lautet:

$$r1\ (ft) = \sum_{m=-\infty}^{+\infty} a_m\ \exp\ (j2\pi mft).$$

Durch Mittelung von r1 (f x t) über n äquidistant im zeitlichen Abstand

$$\Delta t = p/(n \times f)$$

verteilte Abtastpunkte ergibt sich die reduzierte Rippelfunktion rn (ft) zu

$$rn\ (ft) = \frac{1}{n} \sum_{k=0}^{n-1} r1\ (f \times (t + k \times \Delta t))$$

$$= \sum_{m=-\infty}^{+\infty} a_m\ (\frac{1}{n} \sum_{k=0}^{n-1} \exp(j\ 2\pi\ kpm/n))\ \exp\ (j\ 2\pi mft)$$

$$= \sum_{m=-\infty}^{+\infty} a_{m \times n'}\ \exp(j\ 2\pi m \times n'\ ft).$$

Dabei ist n' = n/q, wenn q den größten gemeinsamen Teiler von p und n bezeichnet. Durch die Mittelung werden nur diejenigen Harmonischen nicht unterdrückt, deren Index ein ganzzahliges Vielfaches von n' ist. Die günstigsten Verhältnisse liegen für n = n' vor, d.h. wenn n und p teilerfremd sind.

Es werden die Umlaufzeit tu des Fokus, die Anzahl n der Umläufe und die natürliche Zahl p so gewählt, daß

$$tu = (1 + p/n)tr$$

mit einer geeigneten natürlichen Zahl 1 gilt.

Hierzu ein Beispiel:

Für einen Niederfrequenz-Hochspannungsgenerator 9 kann z.B. tr = 10 ms sein. Wählt man 1 = 5 und p = 1 und n zwischen 10 und 20, so folgt

$$tu = (5 + 1/n)tr \approx 50\ ms.$$

3

Die Bedingung

$$tu = (1 + p/n)\, tr$$

kann durch Frequenzsynchronisation eingehalten werden. Mit $f' = 1/tu$ als Umlauffrequenz ist zu setzen

$$f' = f/(1 + p/n).$$

Über den zentralen Steuerrechner 15 wird die Sollphase vorgegeben, die durch die natürlichen Zahlen 1, p und n charakterisiert ist. Die Synchronisiervorrichtung 18 liefert die Frequenz f', die für die Elektronenstrahlführung auf dem Sollkreis und den Auslesetakt an den Detektorelementen benötigt wird. Alle Spannungen und Ströme sind Schwankungen mit der Frequenz f unterworfen, was in Fig. 1 markiert ist.

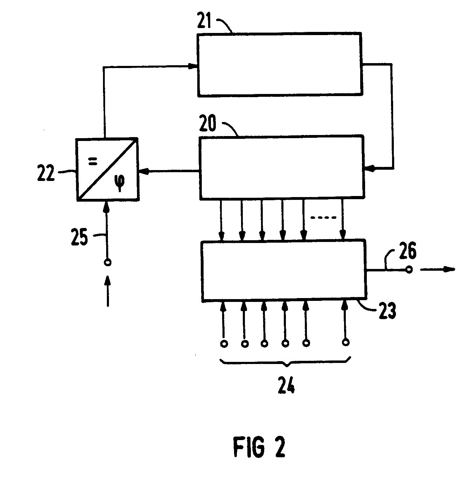

Die Fig. 2 zeigt ein Blockschaltbild einer möglichen Synchronisiervorrichtung 18 mit einem Frequenzteiler 20, einem Oszillator 21, einem Phasen-Spannungswandler 22 und einem Komparator 23, an dessen Eingang 24 eine der Sollphase entsprechende Information liegt. Der Eingang des Phasen-Spannungswandlers 22, an dem die Netzfrequenz liegt, ist mit 25 und der Ausgang mit 26 bezeichnet.

Im vorliegenden Beispiel ist als Netzfrequenz 50 Hz und der Frequenzteiler 20 mit dem Teilerverhältnis 1024 : 1 gewählt, wodurch die Oszillatorfrequenz zu 51.2 kHz festliegt. Es sind aber auch 60 Hz Netzfrequenz und andere Teilerverhältnisse denkbar. Die Synchronisiervorrichtung 18 liefert die Frequenz f' für den Ablenkgenerator 6 und das Datenerfassungssysten 14.

**Patentansprüche**

1. Computertomograph mit einem Röntgenstrahler (1, 3, 5, 7, 8) mit kreisförmigem, eine Meßöffnung (2) umschließendem Anodenbogen (3), auf dem der Fokus zur Durchstrahlung eines in der Meßöffnung (2) liegenden Objektes (11) mit einem fächerförmigen Röntgenstrahlenbündel unter verschiedenen Richtungen mit Hilfe eines Ablenksystems (5, 6) bewegt wird, sowie mit einem kreisförmigen Strahlendetektor (13), der aus einer Reihe von Detektorelementen besteht, deren Meßwerte über ein Datenerfassungssystem (14) einem Rechner (15) zugeführt werden, welcher die Schwächungswerte vorbestimmter Punkte der untersuchten Objektschicht berechnet und deren bildliche Wiedergabe bewirkt, **dadurch gekennzeichnet,** daß das Ablenksystem (5, 6) und das Datenerfassungssystem (14) derart synchronisiert sind, daß bei jedem der n Umläufe des Fokus die Fokusbewegung und das Auslesen der Meßwerte gegenüber dem vorausgegangenen Umlauf relativ zur Rippelperiode der Hochspannung des Röntgenstrahlers (1, 3, 5, 7, 8) geringfügig phasenversetzt erfolgt.

2. Computertomograph nach Anspruch 1, **dadurch gekennzeichnet,** daß der Phasenversatz das p-fache des n ten Teiles der Rippelperiode beträgt, wobei p eine beliebige natürliche Zahl ist.

3. Computertomograph nach Anspruch 2, **dadurch gekennzeichnet,** daß p und n teilerfremd sind.

**Claims**

1. Computer tomography apparatus having an X-ray source (1, 3, 5, 7, 8) with a circular anode arc (3) which defines a measuring aperture (2) and in which on X-raying a subject (11) lying in the measuring aperture (2) the focus is moved with a fan-shaped X-ray beam in different directions with the aid of a deflection system (5, 6), and also having a circular radiation detector (13) which consists of a series of detector elements, the measured values of which are supplied by way of a data-acquisition system (14) to a computer (15) which computes the attenuation values of predetermined points of the layer of the subject examined and effects the image reproduction thereof, characterised in that the deflection system (5, 6) and the data-acquisition system (14) are synchronized in such a way that with each of the n revolutions of the focus the focus movement and the read-out of the measured values is (sic) effected in a slightly phase-shifted manner compared with the preceding revolution relative to the ripple period of the high voltage of the X-ray source (1, 3, 5, 7, 8).

2. Computer tomography apparatus according to claim 1, characterised in that the phase-shift amounts to p-times the nth portion of the ripple period, with p being any natural number.

3. Computer tomography apparatus according to claim 2, characterised in that p and n are relatively prime.

**Revendications**

1. Tomodensitomètre comportant un émetteur radiologique (1,3,5,7,8) possédant un arc anodique (3) de forme circulaire qui entoure une ouverture de mesure (2) et sur lequel le foyer est déplacé de manière à irradier un objet (11) situé dans l'ouverture de mesure (2), avec un faisceau de rayons X en éventail dans différentes directions, à l'aide d'un système de déviation (5,6), ainsi qu'un détecteur de rayonnement de forme circulaire (13), qui est constitué par une série d'éléments détecteurs, dont les valeurs de mesure sont envoyées par l'intermédiaire d'un système d'acquisition de données (14) à un ordinateur (15), qui calcule les valeurs d'affaiblissement de points prédéterminés de la couche examinée de l'objet et réalise leur reproduction sous la forme d'une image, caractérisé par le fait que le système de déviation (5,6) et le système d'acquisition de données (14) sont synchronisés de telle sorte que lors de chacune des n rotations du foyer, par rapport à la rotation précédente, le déplacement du foyer et la lecture des valeurs de mesure sont réalisés avec un léger déphasage vis-à-vis de la période d'ondulation de la haute tension de l'émetteur radiologique (1,3,5,7,8).

2. Tomodensitomètre suivant la revendication 1, caractérisé par le fait que le déphasage est égal à p fois la n-ème partie de la période d'ondulation, p étant un nombre naturel quelconque.

3. Tomodensitomètre suivant la revendication 2, caractérisé par le fait que p et n sont des nombres premiers.

5

FIG 1

FIG 2